(19) Europäisches Patentamt
European Patent Office
Office européen des brevets

(11) Veröffentlichungsnummer: **0 337 198 A2**

(12) **EUROPÄISCHE PATENTANMELDUNG**

(21) Anmeldenummer: 89105584.0

(51) Int. Cl.4: **C07D 249/08 , A01N 43/653**

(22) Anmeldetag: 30.03.89

(30) Priorität: 12.04.88 DE 3812134

(43) Veröffentlichungstag der Anmeldung: 18.10.89 Patentblatt 89/42

(84) Benannte Vertragsstaaten: AT BE CH DE ES FR GB GR IT LI NL SE

(71) Anmelder: BASF Aktiengesellschaft
Carl-Bosch-Strasse 38
D-6700 Ludwigshafen(DE)

(72) Erfinder: Seele, Rainer, Dr.
Leiblstrasse 3
D-6701 Fussgoenheim(DE)
Erfinder: Sauter, Hubert, Dr.
Neckarpromenade 20
D-6800 Mannheim 1(DE)
Erfinder: Kober, Reiner, Dr.
Im Schlittweg 20
D-6701 Fussgoenheim(DE)
Erfinder: Ammermann, Eberhard, Dr.
Sachsenstrasse 3
D-6700 Ludwigshafen(DE)
Erfinder: Lorenz, Gisela, Dr.
Erlenweg 13
D-6730 Neustadt(DE)

(54) **Allyltriazole und diese enthaltende Fungizide.**

(57) Allyltriazole der allgemeinen Formel I

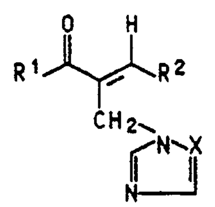

(I)

in welcher $R_1$ und $R_2$ Alkyl, Naphthyl, Biphenyl, Phenyl, Benzylphenyl, Benzyloxyphenyl, Thienyl, Furyl, Pyridyl, Norbornyl, Tetrahydropyranyl, Cycloalkyl oder Cycloalkenyl bedeuten, wobei diese Reste gegebenenfalls substituiert sind,
X, CH oder N bedeutet, sowie deren für Pflanzen verträgliche Säureadditionssalze und Metallkomplexe und diese Verbindungen enthaltende Fungizide.

EP 0 337 198 A2

## Allyltriazole und diese enthaltende Fungizide

Die vorliegende Erfindung betrifft neue Azolverbindungen, Verfahren zu ihrer Herstellung und diese enthaltende Fungizide.

Es ist bekannt, Triazolyl-butenol-Derivate, z.B. das 1-(1,2,4-Triazol-1-yl-methyl)-1-(4-chlorphenyl)-3-(2-chlorphenyl)-prop-2-en-1-ol als Fungizide zu verwenden (EP-52 424). Seine fungizide Wirkung ist jedoch ungenügend.

Es wurde nun gefunden, daß Allyltriazole der allgemeinen Formel I

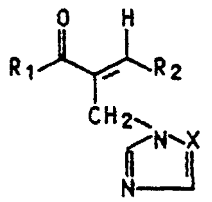

(I)

in welcher $R_1$ und $R_2$ gleich oder verschieden sind und $C_1$-$C_{12}$-Alkyl, Naphthyl, Biphenyl, Phenyl, Benzylphenyl, Benzyloxyphenyl, Thienyl, Furyl, Pyridyl, Norbornyl, Tetrahydropyranyl, $C_3$-$C_{12}$-Cycloalkyl oder $C_3$-$C_{12}$-Cycloalkenyl bedeuten, wobei diese Reste gegebenenfalls durch Halogen, Nitro, Phenoxy, Alkyl, Alkoxy, Amino oder Halogenalkyl mit jeweils 1 bis 4 C-Atomen substituiert sind,
X, CH oder N bedeutet, und deren für Pflanzen verträgliche Säureadditionssalze oder Metallkomplexe eine bessere fungizide Wirkung, insbesondere gegen Getreidekrankheiten, besitzen als bekannte Azolverbindungen.

$R_1$ bzw. $R_2$ bedeuten beispielsweise: $C_1$-$C_{12}$-, insbesondere $C_1$-$C_4$-Alkyl, z.B. Methyl, Ethyl, Isopropyl, n-Propyl, n-Butyl, sec-Butyl, tert.-Butyl, n-Pentyl, Neopentyl, Hexyl, Octyl, Decyl, Dodecyl, Trifluormethyl, Trichlormethyl, Phenyl, Halogenphenyl, 2-Chlorphenyl, 2-Fluorphenyl, 2-Bromphenyl, 3-Chlorphenyl, 3-Bromphenyl, 3-Fluorphenyl, 4-Fluorphenyl, 4-Chlorphenyl, 4-Bromphenyl, 2,4-Dichlorphenyl, 2,3-Dichlorphenyl, 2,5-Dichlorphenyl, 2,6-Dichlorphenyl, 2-Chlor-6-fluorphenyl, Alkoxyphenyl, 2-Methoxyphenyl, 3-Methoxyphenyl, 4-Methoxyphenyl, 2,4-Dimethoxyphenyl, Alkylphenyl, 4-Ethylphenyl, 4-Isopropylphenyl, 4-tert.-Butylphenyl, 4-tert.-Butyloxyphenyl, 2-Chlor-4-fluorphenyl, 2-Chlor-6-methylphenyl, 3,4-Dimethylphenyl, 3-Phenoxyphenyl, 4-Phenoxyphenyl, 3-Nitrophenyl, 4-Nitrophenyl, 3-Aminophenyl, 4-Aminophenyl, 2-Trifluoromethylphenyl, 3-Trifluoromethylphenyl, 4-Trifluoromethylphenyl, 4-Phenoxyphenyl, 4-Benzylphenyl, 4-Benzyloxyphenyl, 2-Thienyl, 3-Thienyl, 2-Furyl, 3-Pyridyl, 4-Pyridyl, Cyclopropyl, Cyclobutyl, Cyclopentyl, Cyclohexyl, Cyclooctyl, Cyclododecyl, Tetrahydropyranyl, 3-Cyclohexenyl, Norbornyl.

Säureadditionssalze sind beispielsweise die Salze mit anorganischen oder organischen Säuren z.B. die Hydrochloride, Bromide, Sulfate, Nitrate, Phosphate, Oxalate oder Dodecylbenzolsulfonate. Die Wirksamkeit der Salze geht auf das Kation zurück, so daß das Anion i.a. beliebig gewählt werden kann. Die erfindungsgemäßen Wirkstoffsalze werden hergestellt durch Umsetzung der Allyltriazole (I) mit den Säuren.

Metallkomplexe der Wirkstoffe I oder ihrer Salze können z.B. mit den Metallen Kupfer, Zink, Zinn, Mangan, Eisen, Kobalt oder Nickel gebildet werden, indem man die Allyltriazole mit entsprechenden Metallsalzen umsetzt, z.B. mit Kupfersulfat, Zinnchlorid, Zinksulfat.

Die Verbindungen der Formel I können z.B. hergestellt werden, indem man eine Verbindung der Formel II

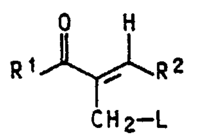

(II)

in welcher $R_1$ und $R_2$ die oben angegebenen Bedeutungen haben und L eine nucleophil substituierbare Abgangsgruppe (OH, Halogen) darstellt, mit einer Verbindung der Formel III

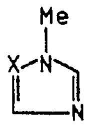 (III)

in der Me ein Wasserstoffatom, ein Metallatom (Na, K) oder eine Trimethylsilylgruppe bedeutet und X die oben angegebene Bedeutung hat, zur Umsetzung bringt.

Die Reaktion a) erfolgt - falls Me ein Wasserstoffatom bedeutet -gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers bei Temperaturen zwischen 10 und 120° C. Zu den bevorzugten Lösungs- und Verdünnungsmitteln gehören Ketone wie Aceton, Methylethylketon oder Cyclohexanon, Nitrile wie Acetonitril oder Propionitril, Alkohole wie Methanol, Ethanol, iso-Propanol, n-Butanol oder Glycol, Ester wie Essigsäureethylester, Essigsäuremethylester oder Essigsäurebutylester, Ether wie Tetrahydrofuran, Diethylether, Dimethoxyethan, Dioxan oder Diisopropylether, Amide wie Dimethylformamid, Dimethylacetamid oder N-Methylpyrrolidon, ferner Dimethylsulfoxid, Sulfolan oder entsprechende Gemische.

Geeignete Basen, die gegebenenfalls auch als säurebindende Mittel bei der Reaktion verwendet werden können, sind beispielsweise Alkalihydroxid wie Lithium-, Natrium- oder Kaliumhydroxid, Alkalicarbonate wie Natrium- oder Casiumcarbonat oder Natrium-, Kalium- oder Cäsiumhydrogencarbonat, Pyridin oder 4-Dimethylaminopyridin. Es können aber auch andere übliche Basen verwendet werden.

Als Reaktionsbeschleuniger kommen vorzugsweise Metallhalogenide wie Natriumjodid, oder Kaliumjodid, quarternäre Ammoniumsalze wie Tetrabutylammoniumchlorid, -bromid, -jodid oder -hydrogensulfat, Benzyltriethylammoniumchlorid oder -bromid oder Kronenether wie 12-Krone-4, 15-Krone-5, 18-Krone-6, Dibenzo-18-Krone-6 oder Dicyclohexano-18-Krone-6 in Frage.

Die Umsetzung wird im allgemeinen bei Temperaturen zwischen 20 und 150° C, drucklos oder unter Druck, kontinuierlich oder diskontinuierlich durchgeführt.

Ist Me ein Metallatom wird die Reaktion a) gegebenenfalls in Gegenwart eines Lösungs- oder Verdünnungsmittels und gegebenenfalls unter Zusatz einer starken anorganischen oder organischen Base bei Temperaturen zwischen -10 und 120° C durchgeführt. Zu den bevorzugten Lösungs- und Verdünnungsmitteln gehören Amide wie Dimethylformamid, Diethylformamid, Dimethylacetamid, Diethylacetamid, N-Methylpyrrolidon, Hexamethylphosphortriamid, Sulfoxide wie Dimethylsulfoxid und Sulfolan.

Geeignete Basen, die gegebenenfalls auch als säurebindende Mittel bei der Reaktion verwendet werden können, sind beispielsweise Alkalihydride wie Lithium-, Natrium- und Kaliumhydrid, Alkaliamide wie Natrium- oder Kalium-triphenylmethyl und Naphthalinlithium, -natrium oder -kalium.

Für die Reaktion b) kommen als Verdünnungsmittel polare organische Lösungsmittel wie Nitrile, z.B. Acetonitril, Sulfoxide, z.B. Dimethylsulfoxid, Formamide, z.B. Dimethylformamid, Ketone, z.B. Aceton, Ether, z.B. Diethylether, Tetrahydrofuran und insbesondere Chlorkohlenwasserstoffe, z.B. Methylenchlorid und Chloroform, in Frage.

Man arbeitet im allgemeinen zwischen 0 und 100° C, vorzugsweise bei 20 bis 80° C. Bei Anwesenheit eines Lösungsmittels wird zweckmäßigerweise beim Siedepunkt des jeweiligen Lösungsmittels gearbeitet.

Die Verbindung II stellt man her, indem man Olefine der Formel IV

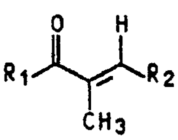 (IV)

nach bekannten Methoden in der Allylposition halogeniert oder oxidiert.

Geeignete Halogenisierungsreagenzien sind N-Chlor- und N-Bromsuccinimid in halogenierten Kohlenwasserstoffen wie Tetrachlorkohlenstoff, Trichlorethan oder Methylenchlorid bei Temperaturen zwischen 20 und 100° C. Zur Allyloxidation verwendet man Perester wie Perbenzoesäure-tert.-butylester oder Peressigsäure-tert.-butylester in Anwesenheit eines Schwermetallsalzes wie z.B. Kupfer-I-chlorid oder Kupfer-I-bromid. Man arbeitet in inerten Lösungsmitteln bei Temperaturen zwischen 10 und 100° C.

Während die so erhaltenen Allylhalogenide II (L = Halogen) gemäß Verfahren a) sofort umgesetzt werden können, überführt man die entsprechenden Allylalkohole II (L = 0H) in reaktive Ester, die dann mit den Verbindungen III gemäß Verfahren a) umgesetzt werden.

Die Darstellung der reaktiven Ester, die mit III umgesetzt werden, erfolgt nach allgemein bekannnten

Methoden (Houben-Weyl-Müller, Methoden der organischen Chemie, Georg Thieme Verlag, Stuttgart, 1955, Band 9, Seiten 388, 663, 671). Solche Ester sind beispielweise Methansulfonsäureester, Trifluormethansulfonsäureester, 2,2,2-Trifluorethansulfonsäureester, Nonafluorbutansulfonsäureester, 4-Methylbenzolsulfonsäureester, 4-Brombenzolsulfonsäureester, 4-Nitrobenzolsulfonsäureester oder Benzolsulfonsäureester.

Die Verbindungen IV lassen sich entsprechend allgemein bekannten Verfahren zur Olefinsynthese (Houben-Weyl-Müller, Methoden der organischen Chemie, Georg Thieme Verlag, Stuttgart, 1972, Bd. V, 1b) herstellen.

Die folgenden Beispiele erläutern die Herstellung der Wirkstoffe.

I. Herstellung der Ausgangsstoffe

Beispiel A

Zu einer Lösung von 50 g 2-Chlorbenzaldehyd in 200 ml Ethanol werden 5,85 g Natriumhydroxid in 40 ml Wasser gegeben. Das Reaktionsgemisch wird auf 10 °C gekühlt und schnell 60 g 4-Chlorpropiophenon zugesetzt, wobei die Temperatur in der Lösung auf 30 bis 40 °C ansteigt. Nach 10stündigem Rühren wird der farblosen Reaktionslösung 200 ml Wasser zugesetzt und die entstandene Emulsion mit Methyl-tert.-butylether ausgeschüttelt. Die organische Phase wird abgetrennt, über Natriumsulfat getrocknet und im Wasserstrahlvakuum eingeengt. Bei der anschließenden Destillation des verbleibenden Rückstandes werden bei 0,25 mbar und 127 °C Übergangstemperatur 84 g (80 %) E-1-(2-Chlorphenyl)-2-(4-chlorbenzoyl)-propen, das aus Methyl-tert.-butylether/n-Hexan kristallisiert werden kann, erhalten. Fp.: 45 - 47 °C.

Beispiel B

84 g E-1-(2-Chlorphenyl)-2-(4-chlorbenzoyl)-propen werden in 200 ml Tetrachlorkohlenstoff gelöst und 52 g N-Bromsuccinimid zugesetzt. Nachdem 0,2 g Azobisisobutyronitril zugegeben wurde, erhitzt man die Reaktionslösung für vier Stunden unter Rückfluß. Das ausgefallene Succinimid wird abfiltriert und das verbleibende Filtrat mit Natriumcarbonat-Lösung (10 %ig) und Wasser gewaschen, über Natriumsulfat getrocknet und eingeengt. Man erhält 102 g (95 %) E-1-(2-Chlorphenyl)-2-(4-chlorbenzoyl)-3-brom-propen.

II. Herstellung der Endprodukte

Beispiel 1

Eine Lösung von 4,9 g Triazol in 100 ml N-Methyl-pyrrolidon wird mit 2,7 g Natriumhydroxid versetzt und für 30 Minuten auf 50 °C erwärmt. Nachdem das Reaktionsgemisch auf Raumtemperatur (20 °C) gekühlt wurde, wird der Lösung 20 g E-1-(2-Chlorphenyl)-2-(4-chlorbenzoyl)-3-brom-propen, das in 100 ml N-Methylpyrrolidon gelöst ist, langsam zugetropft und 12 Stunden bei Raumtemperatur gerührt. Anschließend wird 200 ml Wasser zugegeben und mehrmals mit Methyl-tert.-butylether extrahiert, die organische Phase zweimal mit Wasser gewaschen, über Natriumsulfat getrocknet und eingeengt. Der verbleibende Rückstand wird einer Chromatographie an Kieselgel mit n-Hexan/Essigester = 9:1 unterworfen, wobei 10,8 g (56 %) E-1-(2-Chlorphenyl)-2-(4-chlorbenzoyl)-3-(1,2,4-triazol-1-yl) propen erhalten werden, das aus Methyl-tert.-butylether/n-Hexan kristallisiert werden kann, Fp.: 89-93 °C (Verbindung-Nr. 1).

Entsprechend Beispiel 1 können die in der Tabelle aufgeführten Verbindungen hergestellt werden.

Tabelle

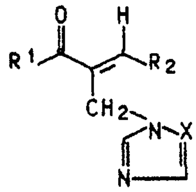

| Bsp. | $R_1$ | $R_2$ | X | Schmp./IR |
|---|---|---|---|---|
| 1 | $4-Cl-C_6H_4$ | $2-Cl-C_6H_4$ | N | 89-93°C |
| 2 | $4-Cl-C_6H_4$ | $3-Cl-C_6H_4$ | N | |
| 3 | $4-Cl-C_6H_4$ | $4-Cl-C_6H_4$ | N | |
| 4 | $4-Cl-C_6H_4$ | $2,4-Cl_2-C_6H_3$ | N | Harz |
| 5 | $4-Cl-C_6H_4$ | $2-F-C_6H_4$ | N | 1651,1501,1272,1137,1015 $cm^{-1}$ |
| 6 | $4-Cl-C_6H_4$ | $4-F-C_6H_4$ | N | |
| 7 | $4-Cl-C_6H_4$ | $4-Br-C_6H_4$ | N | |
| 8 | $4-Cl-C_6H_4$ | $2-Cl-4-F-C_6H_3$ | N | |
| 9 | $4-Cl-C_6H_4$ | $2-CF_3-C_6H_4$ | N | |
| 10 | $4-Cl-C_6H_4$ | $2-OCH_3-C_6H_4$ | N | |
| 11 | $4-Cl-C_6H_4$ | Cyclohexyl | N | |
| 12 | $4-Cl-C_6H_4$ | Cyclopropyl | N | |
| 13 | $4-Cl-C_6H_4$ | 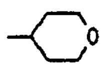 | N | |
| 14 | Phenyl | $3-Cl-C_6H_4$ | N | |
| 15 | Phenyl | $3-Cl-C_6H_4$ | N | |
| 16 | Phenyl | $4-Cl-C_6H_4$ | N | |
| 17 | Phenyl | $2,4-Cl_2-C_6H_3$ | N | 110-112°C |
| 18 | Phenyl | $2-Cl-4-F-C_6H_3$ | N | |
| 19 | Phenyl | $2-F-C_6H_4$ | N | |
| 20 | Phenyl | $4-F-C_6H_4$ | N | |
| 21 | Phenyl | $2-CF_3-C_6H_4$ | N | |
| 22 | Phenyl | Phenyl | N | 1647,1503,1272,1138,1016 $cm^{-1}$ |
| 23 | Phenyl | $4-Br-C_6H_4$ | N | |
| 24 | Phenyl | $2-OCH_3-C_6H_4$ | N | |
| 25 | Phenyl | $3-NO_2-C_6H_4$ | N | |
| 26 | Phenyl | $4-NO_2-C_6H_4$ | N | |
| 27 | Phenyl | $3-NH_2-C_6H_4$ | N | |
| 28 | Phenyl | $4-NH_2-C_6H_4$ | N | |
| 29 | Phenyl | $4-CH_3-C_6H_4$ | N | |
| 30 | Phenyl | 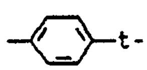-t-Butyl | N | |
| 31 | Phenyl | α-Naphthyl | N | |

Tabelle (Fortsetzung)

| Bsp. | $R_1$ | $R_2$ | X | Schmp./IR |
|---|---|---|---|---|
| 32 | Phenyl | β-Naphthyl | N | |
| 33 | Phenyl | Biphenyl | N | |
| 34 | Phenyl | Cyclopropyl | N | |
| 35 | Phenyl | Cyclopentyl | N | |
| 36 | Phenyl | Cyclohexyl | N | |
| 37 | Phenyl | 3-Cyclohexenyl | N | |
| 38 | Phenyl | Norbornyl | N | |
| 39 | Phenyl | 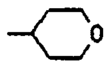 | N | |
| 40 | Phenyl | 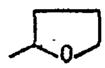 | N | |
| 41 | Phenyl | 3-Pyridyl | N | |
| 42 | $4-F-C_6H_4$ | $2-Cl-C_6H_4$ | N | |
| 43 | $4-F-C_6H_4$ | $3-Cl-C_6H_4$ | N | |
| 44 | $4-F-C_6H_4$ | $4-Cl-C_6H_4$ | N | |
| 45 | $4-F-C_6H_4$ | $2,4-Cl_2-C_6H_3$ | N | |
| 46 | $4-F-C_6H_4$ | $2-Cl-4-F-C_6H_3$ | N | |
| 47 | $4-F-C_6H_4$ | $2-F-C_6H_4$ | N | |
| 48 | $4-F-C_6H_4$ | $4-F-C_6H_4$ | N | |
| 49 | $4-F-C_6H_4$ | $2-CF_3-C_6H_4$ | N | |
| 50 | $4-F-C_6H_4$ | Phenyl | N | |
| 51 | $4-F-C_6H_4$ | $4-Br-C_6H_4$ | N | |
| 52 | $4-F-C_6H_4$ | $2-OCH_3-C_6H_4$ | N | |
| 53 | $4-F-C_6H_4$ | $3-NO_2-C_6H_4$ | N | |
| 54 | $4-F-C_6H_4$ | $4-NO_2-C_6H_4$ | N | |
| 55 | $4-F-C_6H_4$ | $3-NH_2-C_6H_4$ | N | |
| 56 | $4-F-C_6H_4$ | $4-CH_3-C_6H_4$ | N | |
| 57 | $4-F-C_6H_4$ | 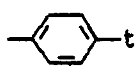t-Butyl | N | |
| 58 | $4-F-C_6H_4$ | α-Naphthyl | N | |
| 59 | $4-F-C_6H_4$ | β-Naphthyl | N | |
| 60 | $4-F-C_6H_4$ | Biphenyl | N | |
| 61 | $4-F-C_6H_4$ | Cyclopropyl | N | |
| 62 | $4-F-C_6H_4$ | Cyclopentyl | N | |
| 63 | $4-F-C_6H_4$ | Cyclohexyl | N | |
| 64 | $4-F-C_6H_4$ | 3-Cyclohexenyl | N | |
| 65 | $4-F-C_6H_4$ | Norbornyl | N | |
| 66 | $4-F-C_6H_4$ | 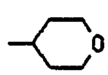 | N | |

Tabelle (Fortsetzung)

| Bsp. | $R_1$ | $R_2$ | X | Schmp./IR |
|---|---|---|---|---|
| 67 | $2,4-Cl_2-C_6H_3$ | $2-Cl-C_6H_4$ | N | |
| 68 | $2,4-Cl_2-C_6H_3$ | $3-Cl-C_6H_4$ | N | |
| 69 | $2,4-Cl_2-C_6H_3$ | $4-Cl-C_6H_4$ | N | |
| 70 | $2,4-Cl_2-C_6H_3$ | $2,4-Cl_2-C_6H_3$ | N | |
| 71 | $2,4-Cl_2-C_6H_3$ | $2-Cl-4-F-C_6H_3$ | N | |
| 72 | $2,4-Cl_2-C_6H_3$ | $2-F-C_6H_4$ | N | |
| 73 | $2,4-Cl_2-C_6H_3$ | $4-F-C_6H_4$ | N | |
| 74 | $2,4-Cl_2-C_6H_3$ | $4-Br-C_6H_4$ | N | |
| 75 | $2,4-Cl_2-C_6H_3$ | $2-CF_3-C_6H_4$ | N | |
| 76 | $2,4-Cl_2-C_6H_3$ | $2-OCH_3-C_6H_4$ | N | |
| 77 | $2,4-Cl_2-C_6H_3$ | $3-NO_2-C_6H_4$ | N | |
| 78 | $2,4-Cl_2-C_6H_3$ | $4-NO_2-C_6H_4$ | N | |
| 79 | $2,4-Cl_2-C_6H_3$ | $3-NH_2-C_6H_4$ | N | |
| 80 | $2,4-Cl_2-C_6H_3$ | $4-NH_2-C_6H_4$ | N | |
| 81 | $2,4-Cl_2-C_6H_3$ | $4-CH_3-C_6H_4$ | N | |
| 82 | $2,4-Cl_2-C_6H_3$ | β-Naphthyl | N | |
| 83 | $2,4-Cl_2-C_6H_3$ | Biphenyl | N | |
| 84 | $2,4-Cl_2-C_6H_3$ | Cyclopropyl | N | |
| 85 | $2,4-Cl_2-C_6H_3$ | Cyclohexyl | N | |
| 86 | $2,4-Cl_2-C_6H_3$ | 3-Cyclohexenyl | N | |
| 87 | $2,4-Cl_2-C_6H_3$ | Norbornyl | N | |
| 88 | $2,4-Cl_2-C_6H_3$ | 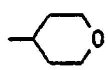 | N | |
| 89 | $2-Cl-C_6H_4$ | $2-Cl-C_6H_4$ | N | |
| 90 | $2-Cl-C_6H_4$ | $4-Cl-C_6H_4$ | N | |
| 91 | $2-Cl-C_6H_4$ | $2,4-Cl_2-C_6H_3$ | N | |
| 92 | $2-Cl-C_6H_4$ | $2-Cl-4-F-C_6H_3$ | N | |
| 93 | $2-Cl-C_6H_4$ | $2-F-C_6H_4$ | N | |
| 94 | $2-Cl-C_6H_4$ | $4-F-C_6H_4$ | N | |
| 95 | $2-Cl-C_6H_4$ | $2-CF_3-C_6H_4$ | N | |
| 96 | $2-Cl-C_6H_4$ | Phenyl | N | |
| 97 | $2-Cl-C_6H_4$ | $2-OCH_3-C_6H_4$ | N | |
| 98 | $2-Cl-C_6H_4$ | $3-NO_2-C_6H_4$ | N | |
| 99 | $2-Cl-C_6H_4$ | $4-NO_2-C_6H_4$ | N | |
| 100 | $2-Cl-C_6H_4$ | $4-NH_2-C_6H_4$ | N | |
| 101 | $2-Cl-C_6H_4$ | $4-CH_3-C_6H_4$ | N | |
| 102 | $2-Cl-C_6H_4$ | β-Naphthyl | N | |

Tabelle (Fortsetzung)

| Bsp. | $R_1$ | $R_2$ | X | Schmp./IR |
|---|---|---|---|---|
| 103 | $2-Cl-C_6H_4$ | Biphenyl | N | |
| 104 | $2-Cl-C_6H_4$ | Cyclopropyl | N | |
| 105 | $2-Cl-C_6H_4$ | Cyclohexyl | N | |
| 106 | $2-Cl-C_6H_4$ | 3-Cyclohexenyl | N | |
| 107 | $2-Cl-C_6H_4$ | Norbornyl | N | |
| 108 | $2-F-C_6H_4$ | $2-Cl-C_6H_4$ | N | |
| 109 | $2-F-C_6H_4$ | $4-Cl-C_6H_4$ | N | |
| 110 | $2-F-C_6H_4$ | $2,4-Cl_2-C_6H_3$ | N | |
| 111 | $2-F-C_6H_4$ | $2-Cl-4-F-C_6H_3$ | N | |
| 112 | $2-F-C_6H_4$ | $2-F-C_6H_4$ | N | |
| 113 | $2-F-C_6H_4$ | $4-F-C_6H_4$ | N | |
| 114 | $2-F-C_6H_4$ | $2-CF_3-C_6H_4$ | N | |
| 115 | $2-F-C_6H_4$ | Phenyl | N | |
| 116 | $2-F-C_6H_4$ | $2-OCH_3-C_6H_4$ | N | |
| 117 | $2-F-C_6H_4$ | $3-NO_2-C_6H_4$ | N | |
| 118 | $2-F-C_6H_4$ | $4-NH_2-C_6H_4$ | N | |
| 119 | $2-F-C_6H_4$ | $4-CH_3-C_6H_4$ | N | |
| 120 | $2-F-C_6H_4$ | β-Naphthyl | N | |
| 121 | $2-F-C_6H_4$ | Biphenyl | N | |
| 122 | $2-F-C_6H_4$ | Cyclopropyl | N | |
| 123 | $2-F-C_6H_4$ | Cyclohexyl | N | |
| 124 | $2-F-C_6H_4$ | 3-Cyclohexenyl | N | |
| 125 | $2-F-C_6H_4$ | Norbornyl | N | |
| 126 | $4-OCH_3-C_6H_4$ | $2-Cl-C_6H_4$ | N | |
| 127 | $4-OCH_3-C_6H_4$ | $4-Cl-C_6H_4$ | N | |
| 128 | $4-OCH_3-C_6H_4$ | $2,4-Cl_2-C_6H_3$ | N | |
| 129 | $4-OCH_3-C_6H_4$ | $2-Cl-4-F-C_6H_3$ | N | |
| 130 | $4-OCH_3-C_6H_4$ | $2-F-C_6H_4$ | N | |
| 131 | $4-OCH_3-C_6H_4$ | $4-F-C_6H_4$ | N | |
| 132 | $4-OCH_3-C_6H_4$ | $2-CF_3-C_6H_4$ | N | |
| 133 | $4-OCH_3-C_6H_4$ | Phenyl | N | |
| 134 | $4-OCH_3-C_6H_4$ | $2-OCH_3-C_6H_4$ | N | |
| 135 | $4-OCH_3-C_6H_4$ | $3-NO_2-C_6H_4$ | N | |
| 136 | $4-OCH_3-C_6H_4$ | $4-NH_2-C_6H_4$ | N | |
| 137 | $4-OCH_3-C_6H_4$ | $4-CH_3-C_6H_4$ | N | |
| 138 | $4-OCH_3-C_6H_4$ | β-Naphthyl | N | |
| 139 | $4-OCH_3-C_6H_4$ | Biphenyl | N | |
| 140 | $4-OCH_3-C_6H_4$ | Cyclopropyl | N | |
| 141 | $4-OCH_3-C_6H_4$ | Cyclohexyl | N | |
| 142 | $4-OCH_3-C_6H_4$ | 3-Cyclohexenyl | N | |

Tabelle (Fortsetzung)

| Bsp. | $R_1$ | $R_2$ | X | Schmp./IR |
|---|---|---|---|---|
| 143 | $4-OCH_3-C_6H_4$ | Norbornyl | N | |
| 144 | $4$-t.-butyl-$C_6H_4$ | $2-Cl-C_6H_4$ | N | |
| 145 | $4$-t.-butyl-$C_6H_4$ | $4-Cl-C_6H_4$ | N | |
| 146 | $4$-t.-butyl-$C_6H_4$ | $2,4-Cl_2-C_6H_3$ | N | |
| 147 | $4$-t.-butyl-$C_6H_4$ | $2-Cl-4-F-C_6H_3$ | N | |
| 148 | $4$-t.-butyl-$C_6H_4$ | $2-F-C_6H_4$ | N | |
| 149 | $4$-t.-butyl-$C_6H_4$ | $4-F-C_6H_4$ | N | |
| 150 | $4$-t.-butyl-$C_6H_4$ | $2-CF_3-C_6H_4$ | N | |
| 151 | $4$-t.-butyl-$C_6H_4$ | Phenyl | N | |
| 152 | $4$-t.-butyl-$C_6H_4$ | $2-OCH_3H_4$ | N | |
| 153 | $4$-t.-butyl-$C_6H_4$ | $3-NO_2-C_6H_4$ | N | |
| 154 | $4$-t.-butyl-$C_6H_4$ | $4-NH_2-C_6H_4$ | N | |
| 155 | $4$-t.-butyl-$C_6H_4$ | $4-CH_3-C_6H_4$ | N | |
| 156 | $4$-t.-butyl-$C_6H_4$ | B-Naphthyl | N | |
| 157 | $4$-t.-butyl-$C_6H_4$ | Biphenyl | N | |
| 158 | $4$-t.-butyl-$C_6H_4$ | Cyclopropyl | N | |
| 159 | $4$-t.-butyl-$C_6H_4$ | Cyclohexyl | N | |
| 160 | $4$-t.-butyl-$C_6H_4$ | 3-Cyclohexenyl | N | |
| 161 | $4$-t.-butyl-$C_6H_4$ | Norbornyl | N | |
| 162 | $3-NO_2-C_6H_4$ | $2-Cl-C_6H_4$ | N | |
| 163 | $3-NO_2-C_6H_4$ | $4-Cl-C_6H_4$ | N | |
| 164 | $3-NO_2-C_6H_4$ | $2,4-Cl_2-C_6H_3$ | N | |
| 165 | $3-NO_2-C_6H_4$ | $2-Cl-4-F-C_6H_3$ | N | |
| 166 | $3-NO_2-C_6H_4$ | $2-F-C_6H_4$ | N | |
| 167 | $3-NO_2-C_6H_4$ | $4-F-C_6H_4$ | N | |
| 168 | $3-NO_2-C_6H_4$ | $2-CF_3-C_6H_4$ | N | |
| 169 | $3-NO_2-C_6H_4$ | Phenyl | N | |
| 170 | $3-NO_2-C_6H_4$ | $2-OCH_3-C_6H_4$ | N | |
| 171 | $3-NO_2-C_6H_4$ | $3-NO_2-C_6H_4$ | N | |
| 172 | $3-NO_2-C_6H_4$ | $4-NH_2-C_6H_4$ | N | |
| 173 | $3-NO_2-C_6H_4$ | $4-CH_3-C_6H_4$ | N | |
| 174 | $3-NO_2-C_6H_4$ | β-Naphthyl | N | |
| 175 | $3-NO_2-C_6H_4$ | Biphenyl | N | |
| 176 | $3-NO_2-C_6H_4$ | Cyclopropyl | N | |
| 177 | $3-NO_2-C_6H_4$ | Cyclohexyl | N | |
| 178 | $3-NO_2-C_6H_4$ | 3-Cyclohexenyl | N | |
| 179 | $3-NO_2-C_6H_4$ | Norbornyl | N | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide

eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 1 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 4 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 5 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 1 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 4 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 5 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 1 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 4 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 5 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N′-propylen-bis-dithiocarbamat),
Zink-(N,N′-propylen-bis-dithiocarbamat),
N,N′-Polypropylen-bis-(thiocarbamoyl)-disulfid;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;
5-Nitro-isophthalsäure-di-isopropylester
heterocyclische Substanzen, wie
2-Heptadecyl-2-imidazolin-acetat,
2,4-Dichlor-6-(o-chloranilino)-s-triazin,
0,0-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,
2-Thio-1,3-dithio-(4,5-b)-chinoxalin,

1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,
N-Dichlorfluormethylthio-N′,N′-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydroazano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilin,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan ,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N′-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol, sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2′-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3--chlor-2-aminopyridin,
1-((bis-(4-Fluorphenyl)-methylsilyl)-methyl)-1H-1,2,4-triazol.

Anwendungsbeispiele

Als Vergleichswirkstoff wurde 1-(1,2,4-Triazol-1-ylmethyl)-1-(4-chlorphenyl)-3-(2-chlorphenyl)-prop-2-en-1-ol (A) - bekannt aus EP 52 424 -benutzt.

Anwendungsbeispiel 1

Wirksamkeit gegen Plasmopara viticola

Blätter von Topfreben der Sorte "Müller Thurgau" wurden mit wäßriger Spritzbrühe, die 80 % (Gew.%) Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24° C und anschließend für 5 Tage in einem Gewächshaus mit Temperaturen zwischen 20 und 30° C aufgestellt. Nach dieser Zeit wurden die Pflanzen zur Beschleunigung des Sporangienträgerausbruches abermals für 16 Stunden in der feuchten Kammer aufgestellt. Dann erfolgte die Beurteilung des Ausmaßes des Pilzausbruches auf den Blattunterseiten.

Das Ergebnis zeigt, daß die Wirkstoffe 1, 4 und 5 bei der Anwendung als 0,05 %ige Spritzbrühe eine bessere fungizide Wirkung (90 %) zeigen als der bekannte Wirkstoff A (40 %).

## Ansprüche

1. Allyltriazole der allgemeinen Formel I

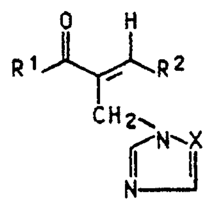

(I)

in welcher $R_1$ und $R_2$ gleich oder verschieden sind und $C_1$-$C_{12}$-Alkyl, Naphthyl, Biphenyl, Phenyl, Benzylphenyl, Benzyloxyphenyl, Thienyl, Furyl, Pyridyl, Norbornyl, Tetrahydropyranyl, $C_3$-$C_{12}$-Cycloalkyl oder $C_3$-$C_{12}$-Cycloalkenyl bedeuten, wobei diese Reste gegebenenfalls durch Halogen, Nitro, Phenoxy, Alkyl, Alkoxy, Amino oder Halogenalkyl mit jeweils 1 bis 4-C-Atomen substituiert sind,
X, CH oder N bedeutet, sowie deren für Pflanzen verträgliche Säureadditionssalze und Metallkomplexe.

2. Verbindungen der Formel I gemäß Anspruch 1, worin $R_1$ und $R_2$ die Phenylgruppe bedeutet, welche unsubstituiert ist oder durch einen oder zwei der Substituenten Fluor, Chlor, Brom oder Trifluormethylgruppe substituiert ist.

3. Verbindungen der Formel I gemäß Anspruch 1, worin die Substituenten in den Resten $R_1$ und $R_2$ Fluor oder Chlor bedeuten.

4. Verfahren zur Herstellung der Allyltriazole der Formel I gemäß Anspruch 1, dadurch gekennzeichnet, daß man eine Verbindung der Formel II

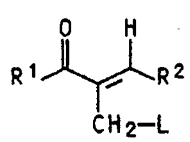

(II)

in welcher $R_1$ und $R_2$ die oben angegebenen Bedeutungen haben und L eine nucleophil substituierbare Abgangsgruppe darstellt, mit einer Verbindung der Formel III

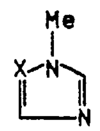

(III)

in der Me ein Wasserstoffatom, ein Metallatom oder eine Trimethylsilylgruppe bedeutet und X die oben angegebene Bedeutung hat, zur Umsetzung bringt.

5. Fungizides Mittel, enthaltend ein Allyltriazol der allgemeinen Formel I

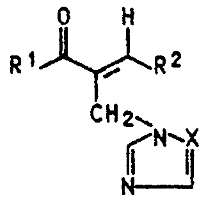

(I)

in welcher $R_1$ und $R_2$ gleich oder verschieden sind und $C_1$-$C_{12}$-Alkyl, Naphthyl, Biphenyl, Phenyl, Benzylphenyl, Benzyloxyphenyl, Thienyl, Furyl, Pyridyl, Norbornyl, Tetrahydropyranyl, $C_3$-$C_{12}$-Cycloalkyl oder $C_3$-$C_{12}$-Cycloalkenyl bedeuten, wobei diese Reste gegebenenfalls durch Halogen, Nitro, Phenoxy, Alkyl, Alkoxy, Amino oder Halogenalkyl mit jeweils 1 bis 4-C-Atomen substituiert sind,
X, CH oder N bedeutet, oder sein für Pflanzen verträgliches Säureadditionssalz oder Metallkomplex und einen inerten Zusatzstoff.

6. Verfahren zur Bekämpfung von Pilzen, dadurch gekennzeichnet, daß man eine fungizid wirksame Menge eines Allyltriazols der allgemeinen Formel I

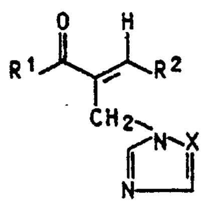

(I)

in welcher $R_1$ und $R_2$ gleich oder verschieden sind und $C_1$-$C_{12}$-Alkyl, Naphthyl, Biphenyl, Phenyl, Benzylphenyl, Benzyloxyphenyl, Thienyl, Furyl, Pyridyl, Norbornyl, Tetrahydropyranyl, $C_3$-$C_{12}$-Cycloalkyl oder $C_3$-$C_{12}$-Cycloalkenyl bedeuten, wobei diese Reste gegebenenfalls durch Halogen, Nitro, Phenoxy, Alkyl, Alkoxy, Amino oder Halogenalkyl mit jeweils 1 bis 4-C-Atomen substituiert sind,
X, CH oder N bedeutet, oder sein für Pflanzen verträgliches Säureadditionssalz oder Metallkomplex auf die Pilze oder auf durch Pilzbefall bedrohte Materialien, Flächen, Pflanzen oder Saatgüter einwirken läßt.

7. 1-(2-Chlorphenyl)-2-(4-chlorbenzoyl)-3-(1,2,4-triazol-1-yl)-prop-1-en.

8. 1-(2,4-Dichlorphenyl)-2-(4-chlorbenzoyl)-3-(1,2,4-triazol-1-yl)-prop-1-en.

9. 1-(2-Fluorphenyl)-2-(4-chlorbenzoyl)-3-(1,2,4-triazol-1-yl)-prop-1-en.